(19) Europäisches Patentamt
European Patent Office
Office européen des brevets

(11) **EP 4 124 314 A1**

(12) **EUROPEAN PATENT APPLICATION**

(43) Date of publication:
**01.02.2023 Bulletin 2023/05**

(21) Application number: **21187691.7**

(22) Date of filing: **26.07.2021**

(51) International Patent Classification (IPC):
***A61B 34/30*** (2016.01)   ***A61B 34/00*** (2016.01)
***A61B 1/00*** (2006.01)

(52) Cooperative Patent Classification (CPC):
**A61B 34/73; A61B 1/00158;** A61B 34/20;
A61B 34/30; A61B 34/72; A61B 2034/732

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Max-Planck-Gesellschaft zur
Förderung der
Wissenschaften e.V.
80539 München (DE)**

(72) Inventors:
• **Son, Donghoon
70569 Stuttgart (DE)**
• **Ugurlu, Musab
70569 Stuttgart (DE)**
• **Blümer, Peter
70569 Stuttgart (DE)**
• **Sitti, Metin
70569 Stuttgart (DE)**

(74) Representative: **Richardt Patentanwälte PartG
mbB
Wilhelmstraße 7
65185 Wiesbaden (DE)**

(54) **MAGNETIC TRAP SYSTEM**

(57)   The present disclosure relates to a magnetic trap system (1000) comprising:
- a microscopic device (300), comprising a principal axis extending in a longitudinal direction;
- a trap (100) for magnetically confining the microscopic device in a confinement region (CR);
- a receptable zone (RZ) for receiving biological mattermatter (400, 800), the receptable zone (RZ) comprising the confinement region (CR);
- a mechanical device (200) for providing a relative movement between the receptable zone (RZ) and the microscopic device (300);
wherein the trap (100) is hollow about a longitudinal axis (A), comprises the receptable zone, and provides a magnetic field gradient configured to confine the microscopic device to the confinement region (CR) of the trap (100);
wherein the orientation of the magnetic field in the confinement region (CR) is to align the microscopic device in the confinement region (CR) with the longitudinal axis (A) of the confinement region.

Fig. 2A

**Description**

BACKGROUND

The present invention relates to the field of navigating a microscopic device (such as a remotely and wirelessly controllable milli-scale robot) biological matter, and specifically relates to a respective method and a system for performing the method and a computer program product.

In the last decade, some remarkable progress has been made in creating small-scale medical robots which can operate inside of the human body. The main benefit offered by such miniature robots is their small size, since they do not require large openings to invasively access many parts of a human body, thereby minimizing surgical trauma. Yet, many challenges also arise from the small size of these milli-scale-robots due to the difficulty to place on-board robotic components, such as actuators, sensors, power source, computation, communication, and control electronics, into the tiny robot body. The approach of using wirelessly operated micro-scale robots has been progressing notably in the recent decade. Namely, with remote actuation principles, such as those involving magnetic fields, acoustic waves and light, many wireless small-scale robots have been used for navigation and for performing medical functions inside the body.

As the human body is composed of mostly soft matters, enabling the small-scale mobile robots to navigate inside not only in fluids but also in such soft matters is important for being able to access some hard-to-reach parts of the human body and treat diseases therein. For example, a robot that can navigate inside the brain could treat a brain cancer without the need for even creating (large) surgical openings in skull and brain matters. Recent attempts enable remotely-actuated miniature robots to navigate inside matters have aimed for blood-clot removal, matter drilling, and navigation in the brain. However, these approaches were limited to using a simple one-dimensional actuation and a low control and localization bandwidth with possible control instability issues caused by strong magnetic forces. Creating strong magnetic forces to penetrate matters has been considered, but such strong magnetic forces may result in the magnetic robot quickly heading towards the magnetic source during a surgical operation when the localization and control systems are not extremely well designed with quick response times. Hence, there would appear to be a high safety risk involved with medical magnetic robot systems.

SUMMARY

Various embodiments provide a magnetic trap system and a method for navigating a microscopic device in biological matter (e.g. biological tissue), as described by the subject matter of the independent claims. Advantageous embodiments are described in the dependent claims. Embodiments of the present invention can be freely combined with each other if they are not mutually exclusive.

In one aspect, the invention relates to a magnetic trap system. The magnetic trap system comprises:

a microscopic device, comprising a principal axis extending in a longitudinal direction;

a trap for magnetically confining the microscopic device in a confinement region;

a receptable zone within the trap for receiving biological matter, the receptable zone comprising the confinement region;

a mechanical device for providing a relative movement between the receptable zone and the microscopic device;

wherein the trap is hollow about a longitudinal axis and comprises the receptable zone;

wherein the trap is configured to provide a magnetic field gradient configured to confine the microscopic device to the confinement region of the trap; and

wherein the orientation of the magnetic field in the confinement region of the trap is configured to align the principal axis of the microscopic device in the confinement region with the longitudinal axis of the confinement region or with an axis that does not deviate more than 30°, preferably not more than 15°, from the longitudinal axis of the confinement region.

The confinement region may thus be defined as the region in which the above criteria are fulfilled, namely the confinement of the microscopic device (especially with a magnetic gradient profile which in the longitudinal direction has zero gradient and changes sign along the longitudinal direction (or" crosses axis") in the center of the CR), and said alignment of the principal axis of the microscopic device.

This may offer a unique way of navigation in plural dimensions, depending on the degrees of freedom offered by the mechanical device. The microscopic device may in particular be a robot for providing diagnostic data of biological matter into which the robot may be inserted (such as local and controlled imaging contrast agent delivery, visual camera monitoring, and biopsy (retrieving matter or liquid samples)). In such matter, alternatively or in addition to a diagnosis, a medical treatment of matter portions may further take place by means of the robot. Such medical treatment may include cauterization, neural recording and/or stimulation, puncturing a specific matter or membrane, removing a clot or matter locally, hyperthermia, cargo (e.g., drugs, stem cells, genes, imaging agents, RNA, proteins, biological markers, radioactive agents, biological cells) delivery, and embolization in brain and other matters.

The trap may magnetically confine the microscopic device in the confinement zone by an interaction of one or more magnets of the trap and one or more magnets of the microscopic device. The magnets of the trap may be permanent magnets or electromagnetic magnets, specifically superconducting magnets. A magnet of the microscopic device may be an NdFeB magnet such as available from Webcraft GmbH, Gottmadingen, Germany.

The confinement of the microscopic device in the trap may serve to have a well-defined location of the microscopic device with respect to the trap, namely in the hollow space of the trap. If the microscopic device is moved by the mechanical device relative to the receptable zone of the trap, there may be a counterforce exerted onto the microscopic device, urging it always back to the confinement zone.

If the microscopic device is inserted into an object such as biological matter and moved together with the matter relative to the trap, then the counterforce may act upon the microscopic device only, but not onto the matter, which leads to a relative motion of the microscopic device in the interior of the matter, i.e., a navigation in 3D space. Such motion may specifically be achieved without an active control of units of the microscopic device.

The relative movement of receptable zone and microscopic device may be achieved by moving the microscopic device, e.g., with a mechanical stage arrangement. Such mechanical stage arrangement may comprise one or two linear translation stages such as model LTS300/M from Thorlabs Inc., Newton NJ, USA, and may further comprise a stepper motor such as model NEMA 17-01, Neukirchen-Vluyn, Germany, for rotational movement. The overall system may be driven by Robot Operation system (ROS Melodic) which runs in Linux operating system (Ubuntu 18.04).

An operator may modulate control inputs to the mechanical device through a wireless gamepad of, e.g., model F710, Logitech, Newark CA, USA. As an alternative to moving the microscopic device with respect to a stationary trap, one may move the magnetic trap by suitable means. The latter may be the more comfortable solution if biological matter including the microscopic device is matter of a living subject (such as a human's brain). Then, the living object does not need to be moved with respect to the ground in the operating room. Further alternatively, both the trap and the microscopic device may be movable.

The feature of aligning the principal axis of the microscopic device in the confinement zone may serve to avoid that the microscopic device is able to radially "escape" from the confinement zone due to radial forces. By aligning the principal axis with the longitudinal axis of the confinement region (and thus perpendicular to the radial direction), the largest surface area of the microscopic device will be oriented in the radial direction. Thus, the (vast) majority of the surface of the robot (preferably more than 70%, more preferably 80% of the surface of the robot) has a surface normal which is perpendicular to the longitudinal direction.

As a consequence, even though, small radial magnetic forces may act onto the microscopic device in the confinement region, since the vast majority of the surface area of the robot is perpendicular to this radial direction of force, this large surface area of the robot results in a high level of friction with the matter in which the robot is received. As a result, the robot could experience such a high resistance due to this large friction surface that no undesired radial movement takes place in the matter despite the radial magnetic forces. Such a radial movement could else lead to harming the biological matter in which the microscopic device may be placed.

The aligning may as well stabilize the movement of counteraction to the movement caused by the mechanical device. In other words, due to the aligning, the movement of the microscopic device in the trap basically takes place only along the trap's longitudinal axis, with the microscopic device having a well-defined orientation as to its principal axis. The confinement region may thus further be defined as the region in which any forces acting perpendicularly (i.e. radially) to the longitudinal direction are smaller than a predefined value, e.g. smaller than a maximum potential friction force between the microscopic device and the matter in the radial direction.

In an embodiment, the microscopic device has a main tubular, preferably cylindrical, body and a tip, preferably conical tip, that extends from the main body. This may enhance the ability of the microscopic device to move in biological matter. The tip acts to reduce a mechanical resistance of the matter, specifically when configured as a conical tip. The body stabilizes the motion, and specifically, a cylindrical body may most easily slip in the matter without any additional mechanical resistance.

In an embodiment, the microscopic device has an aspect ratio of its overall length to a diameter of the main body of between 0.1. and 1000, preferably of between 0.5 and 5, more preferably of between 1.5 and 3.5, still more preferably of between 2.3 and 2.8. This may be suitable dimensions for the microscopic device to move forward and to be less inclined to be subject of radial movement, and may assist in aligning the microscopic device in the confinement region of the trap.

In an embodiment, the microscopic device has a length of between 0.1 and 100 mm, preferably of between 0.75 and 4 mm and a width or diameter of between 0.001 and 5.0 mm, preferably of between 0.25 and 1.8 mm. That may fit to biological matter, for example to typical diameters of arteries and veins in a brain.

In an embodiment, the microscopic device is configured as a robot for medical or surgical treatment. Hence, since the system provides for a perfect navigation in biological matter, this may be matter of a living object to be treated.

In an embodiment, the trap comprises a plurality of permanent magnets adapted for providing the magnetic field. For instance, between 60 and 145 magnets, e.g., between 90 and 100 magnets, such as 95 permanent magnets may be employed. Alternatives may be or include electromagnetic or superconducting magnets. Electromagnets may have the benefit that the electromagnets may be excited to produce their magnetic field only when the matter has already been placed in the confinement region. Thus, this may facilitate introducing the matter without an external magnetic field into the confinement region.

In an embodiment, the trap is configured to provide a magnetic field that has at least one of the following properties:

The magnetic field vectors in the center of the confinement region are parallel to the longitudinal axis or are parallel to an axis deviating not more than 30°, preferably not more than 15°, from the longitudinal axis,

the magnetic field strength parallelly to the longitudinal axis increases from both the left-hand and right-hand borders of the receptable zone, where it has preferably a value of between 1 mT and 100 mT, preferably of between 50 mT and 60 mT, to a value defined in the confinement region,

the value of the magnetic field strength in the confinement region is between 1 mT and 500 mT, preferably between 140 mT to 180 mT,

the magnetic field strength radially increases from the center of the confinement region from a minimum value to a value not more than 15 % of the minimum value,

the magnetic field strength increases radially from the center of the confinement region to a maximum value of between 10 mT and 500 mT, preferably of between 180 mT to 200 mT,

parallel to the longitudinal axis the magnetic field gradient in the confinement region of the trap has a value of between zero in the center of the confinement region and between 0.1 and 30 T/m, preferably between 5 and 10 T/m, more preferably between 6 and 8 T/m, in the immediate vicinity of the border of the confinement region,

the magnetic field gradient at the border of an attraction region has an absolute value of more than 5 T/m, preferably of more than 6.5 T/m,

the absolute value of the magnetic field gradient inside of the confinement region monotonically decreases, preferably strictly monotonically decreases, still more preferably linearly decreases, from a border of the confinement region to a minimum value in the center of the confinement region.

These values have proved to be useful and to assist in having the microscopic device stably navigate. Namely, a) may assist to stabilize the microscopic device in the trap. b) may help to suitable insert the microscopic device into the matter and the matter with the microscopic device into the trap. c) may assist to stably confine the microscopic device in the confinement region. d) may help to align the microscopic device, e) to avoid its escaping to the magnets radially outside of the confinement region. f) may lead to provide an optimum dipole moment onto a magnetic dipole containing microscopic device. g) may be provided for inserting the microscopic device into the matter. h) may be equivalent to a square function describing the magnetic field strength in dependence of position on the longitudinal axis. This may help to provide for smoothly forcing the microscopic device into the trap's confinement region.

In an embodiment, the trap may be configured to provide a first magnetic field and wherein the microscopic device is configured to provide a second magnetic field, wherein the first and second magnetic fields include at least one of the following properties:

the absolute value of the radial force magnetically exerted onto the microscopic device in the confinement region is less than 10 mN, preferably less than 8 mN, more preferably less than 4 mN, still more preferably less than 2 mN.

The absolute value of the axial force magnetically exerted onto the microscopic device parallel to the longitudinal axis in the confinement region is in between 0 in the center of the confinement region and F in the immediate vicinity of the border of the confinement region, F being in between 4-24 mN, preferably 8 — 18 mN, more preferably 12 — 16 mN. may be useful for minimizing movement of the microscopic device in the confinement region. b) may serve to act onto the microscopic device to always force it (back) into the center of the confinement region.

In an embodiment, the trap and the microscopic device are matched to each other in that the confinement region has a length of between 1 times to 500 times, preferably between 10 times to 30 times, more preferably between 15 times and 25 times the length of the microscopic device along its principal axis and/or in that the confinement region has a width of between1 times to 500 times, preferably 10 times and 20 times, more preferably between 13 times and 18 times the width (such as a diameter) of the microscopic device perpendicular to its principal axis. This embodiment enables to most stably align the microscopic device in the trap due to suitable magnetic fields.

In an embodiment, the system comprises an imaging device, preferably an X-ray fluoroscopic or ultrasound imaging device, adapted for monitoring the microscopic device in the confinement region.

In an embodiment thereof, the system includes a controller of the mechanical device for performing the relative movement between the receptable zone and the microscopic device, wherein the controller is configured to receive image data of images taken by the imaging device, to analyze the data, and to control the mechanical device in dependence of the data and a result of the analysis.

In an embodiment, the relative movement provided by the mechanical device (200) is anyone of a longitudinal motion, a radial motion, and a rotation with respect to the longitudinal axis (A) of the trap (100). This allows for a multiplicity of degrees of freedom (e.g., of up to 5), thus facilitating navigation.

In another aspect, the invention relates to a method of navigating a microscopic device in biological matter, e.g. tissue, the method comprising:
providing a magnetic trap system, the magnetic trap system comprising:

a microscopic device, comprising a principal axis extending in a longitudinal direction;

a trap for magnetically confining the microscopic device in a confinement region;

a receptable zone for receiving biological matter, the receptable zone comprising the confinement region;

a mechanical device for providing a relative movement between the receptable zone and the microscopic device;

wherein the trap is hollow about a longitudinal axis and comprises the receptable zone;

wherein the trap is configured to provide a magnetic field gradient configured to confine the microscopic device to the confinement region of the trap; and

wherein the orientation of the magnetic field in the confinement region of the trap is configured to align the principal axis of the microscopic device in the confinement region with the longitudinal axis of the confinement region or with an axis that does not deviate more than 30°, preferably not more than 15°, from the longitudinal axis of the confinement region.

The method thus provides the advantages of the magnetic trap system explained above.

In an embodiment, the method further comprises positioning the biological matter in the receptable zone, wherein the microscopic device may have been inserted into the matter, and further comprises operating the mechanical device, which may cause navigation of the microscopic device in the matter.

In an embodiment, the method comprises providing an imaging device, preferably an X-ray fluoroscopic imaging device, adapted for monitoring the microscopic device in the confinement region, the method further comprising receiving a navigation path, the navigation path describing a desired relative movement of the microscopic device relative to the biological matter, wherein operating the mechanical device for performing the relative movement between the receptable zone and the microscopic device is performed in order to cause the microscopic device to undergo navigation in accordance with the navigation path in the biological matter, the method further comprising during the navigation:

receiving image data of images taken by the imaging device from the confinement region,

receiving a desired spatial location of the microscopic device relative to the biological matter in accordance with the navigation path,

analyzing the data for obtaining an actual spatial location of the microscopic device relative to the biological matter, and in case of a mismatch between the actual location and the desired location, control the mechanical device in dependence of the data and a result of the analysis for correcting the actual spatial location of the microscopic device, the correcting resulting in a matching of the actual spatial location of the microscopic device with the desired spatial location. Hence, imaging may suitably assist in providing an optimum navigation along a desired navigation path.

In an embodiment, providing the trap comprises providing a magnetic field which orients toward the confinement region in order to make the microscopic device head toward the center of the confinement region, thus attracting the microscopic device, as desired.

The magnetic field of the trap and the microscopic device may be adapted to the biological matter such that in the confinement region the radial magnetic force acting onto the microscopic device is smaller or equal than the friction force acting between the matter and the microscopic device in the radial direction, while in the longitudinal direction the magnetic force acting onto the microscopic device is larger than the friction force acting between the matter and the microscopic device. E.g., the radial magnetic force acting onto the microscopic device may be to a factor of between 0.25 and 0.9, preferably of between 0.7 and 0.8, smaller or equal than the friction force acting between the matter and the microscopic device in the radial direction, and the magnetic force acting onto the microscopic device longitudinal direction is to a factor of between 1.1 and 1.8, preferably of between 1.2 and 1.3, larger than the friction force acting between the matter and the microscopic device. These values may provide for the microscopic device remaining stable in the confinement region.

In general, the stable confinement region may be expanded by increasing the radial drag coefficient, $c_r$ and decreasing the axial drag coefficient, $c_a$, of the microscopic device in the matter. Additionally, small radial drag coefficient and large axial drag coefficient can make the confinement region very small so that the robot can become easily unstable. Here, the drag coefficients are the functions of the microscopic device shape and the surrounding matter, which should be carefully chosen to maximize the stable region by increasing the radial drag and by minimizing the axial drag. Note that these drag coefficients could be experimentally measured by calculating the slope of resistance-penetration speed graphs.

Resistance-penetration speed graphs are known in the art. The slope of the resistance-penetration speed graphs may be obtained by linear regression. Specifically, measured data of the resistance-penetration speed experiments can be fitted into the linear polynomial where the coefficient of the terms, the "speed term" becomes the drag coefficient.

In an embodiment, the shape of the microscopic device and the magnetic field strength in the interior portion of the trap are matched to the type of biological matter to be navigated in, specifically matched to a viscosity of such matter, in a manner that the microscopic device when located in such matter in the interior portion of the trap does not damage the matter. The latter is a desired effect to be fulfilled.

In an embodiment, the microscopic device is imaged during navigation and wherein the mechanical device is at least in part automatically operated by means of a controller for ensuring that the mechanical device follows a predetermined path in the biological matter. Such automation may lead to higher reliability of the method.

In an embodiment, the method may comprises providing the trap, the trap comprising a plurality of magnets, wherein the providing comprises determining the relative arrangement of the magnets to each other, the determining being performed employing a numerical nonlinear optimization solver employing a magnetic dipole model. Such features ensure that features of the kind explained above at a) to 1) may be defined and implemented.

It has to be noted that the above-described system and method may also be applied to other matter besides biological matter, like any kinds of fluids or e.g. viscous material.

In another aspect, there is provided a computer program product, in particular a computer readable medium, the computer program product carrying computer executable code for execution by a processor controlling the system of claim 1, wherein execution of the instructions causes the processor to control the mechanical device for performing the relative movement between the receptable zone and the microscopic device.

BRIEF DESCRIPTION OF THE SEVERAL VIEWS OF THE DRAWINGS

In the following embodiments of the invention are explained in greater detail, by way of example only, making reference to the drawings in which:

Fig. 1 schematically illustrates the overall system of an inventive embodiment.

Fig. 2A illustrates an example of a magnetic trap 100 for magnetically confining a microscopic device in accordance with the present subject matter, in a front-top perspective view.

Fig. 2B illustrates the example of the trap 100 shown in Fig. 1A, in a lateral view.

Fig. 3 illustrates an example of an entire magnetic trap system 1000 with the trap, 100 shown in Figs. 2A and 2B, a mechanical stage 200, and a matter phantom (with robot), in accordance with the present subject matter.

Fig. 4 illustrates an example of the force profile desired for a magnetic trap in accordance with the present subject matter.

Fig. 5A illustrates an example of the magnetic field distribution in a magnetic trap of the kind shown in Figs. 2A and 2B, which magnetic trap provides the force profile of Fig. 4, in accordance with the present subject matter.

Fig. 5B illustrates an example of the magnetic force distribution in a magnetic trap of the kind shown in Figs. 2A and 2B, which implements the force profile of Fig. 3, in accordance with the present subject matter.

Fig. 6 illustrates an example of the shape of an exemplary robot that is able to be used in the magnetic trap system of Fig. 3, in accordance with the present subject matter.

Fig. 7A illustrates how the magnetic field distribution of a magnetic trap as in Fig, 4A confines the robot of Fig. 6 in the magnetic trap's confinement region, in accordance with the present subject matter.

Fig. 7B illustrates how the magnetic force distribution of a magnetic trap as in Fig. 4B aligns the robot of Fig. 6 in the magnetic trap's confinement region, in accordance with the present subject matter.

Fig. 8 illustrates theoretical movements the exemplary robot may have.

Fig. 9 illustrates how a robot in the confinement region of the magnetic trap of Fig. 2A/2B can be monitored, in accordance with the present subject matter.

Fig. 10 illustrates a brain to explain the steps of a method of navigating a robot therein, and method of treating the brain (not claimed herein), in accordance with the present subject matter.

Fig. 11 is a flow diagram that illustrates the steps of a method of medically or surgically treating a subject (method not claimed herein) by navigating a robot in the subject's matter, in accordance with the present subject matter.

Fig. 12 shows a sequence of fluoroscopic images illustrating navigating of a robot in biological matter.

DETAILED DESCRIPTION

The descriptions of the various embodiments of the present invention will be presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

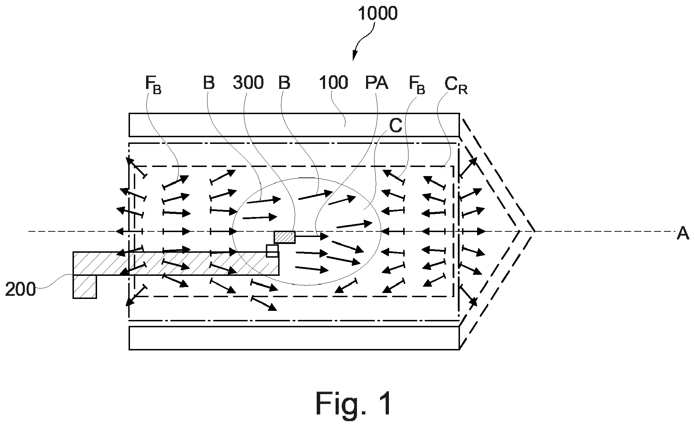

**Figure 1** illustrates a magnetic trap system 1000 comprising a microscopic device 300, comprising a principal axis PA, a trap 100 for magnetically confining the microscopic device 300 in a confinement region CR, a receptable zone RZ for receiving biological matter, the receptable zone RZ comprising the confinement region CR, and a mechanical device 200 for providing a relative movement between the receptable zone RZ and the microscopic device 300.

The trap 100 is hollow about a longitudinal axis A and comprises the receptable zone RZ, and is further configure to provide a magnetic field gradient $F_B$, configured to confine the microscopic device to the confinement region of the trap. The orientation of the magnetic field B in the confinement region CR is configured to align the principal axis PA of the microscopic device in the confinement region of the trap with the longitudinal axis A of the confinement region CR or with an axis that does not deviate more than 10° from the longitudinal axis A of the confinement region CR.

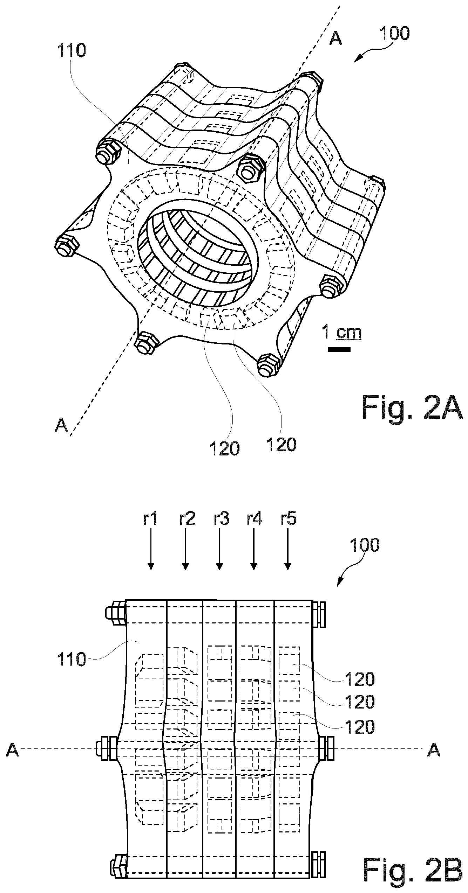

**Figures 2A and 2B** illustrate an example of a magnetic trap 100 in accordance with an example of the present disclosure, in different views. The magnetic trap 100 is in its general shape six-fold rotationally symmetric about a central longitudinal axis A. Other shapes might be possible, including rotationally symmetric shapes with a three-fold, a four-fold, a five-sold, a seven-fold rotational symmetry, or the like. The architecture of the magnetic trap is inspired by Halbach and Aubert arrays. The magnetic trap is here hollow and is about 6 cm long in the direction of the central longitudinal axis A. This is suited to navigate a robot in rather small objects inside of the hollow space. If one were to navigate a robot in a living human's brain, the dimensions would have to be scaled up, e.g. by a factor of five or more.

The magnetic trap 100 may include five rows, or layers, as indicated at r1, r2, r3, r4, and r5 in Fig. 1B. Different numbers of rows than five are possible (two, three, four, six, ...), and due to symmetry reasons preferably unequal numbers (three, seven, nine, eleven, ...).

Each row may include a casing body 110 of plastics material that has been formed by 3D printing, and each such casing body 100 may bear an array of permanent magnets such as indicated in Figs. 2A and 2B at 120. The permanent magnets 120 may be each of identical shape and material. There may be at least five magnets per array, and in the present example, about 15 to 25 are used per array such that the five arrays in rows r1, r2, r3, r4, and r5 together include about 100 permanent magnets (e.g., 95).

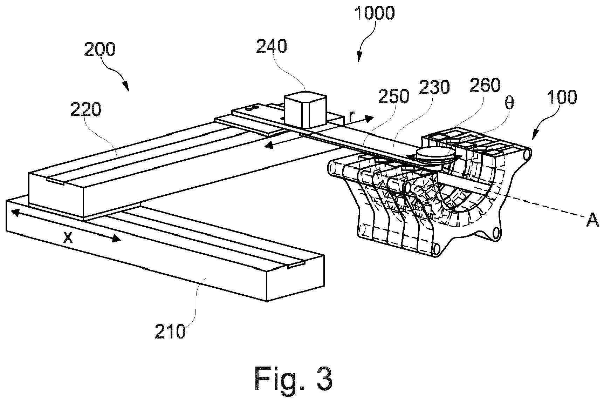

**Figure 3** illustrates an example of the magnetic trap system 1000 as a whole. The magnetic trap of Figs. 2A and 2B is here shown in a broken-away view illustrating only its lower half. An example of a mechanical device is shown as a mechanical stage 200. The mechanical stage 200 may comprise a lower linear stage 210 and an upper linear stage 220 that bears a platform 230. The upper linear stage 220 may be moved by the lower linear stage 210 in a (horizontal) direction parallel to the longitudinal axis A of the magnetic trap 100. The platform 230 may be moved by the upper linear stage 220 in a (preferably horizontal) direction perpendicular to the longitudinal axis A of the magnetic trap 100.

The platform 230 may bear a stepper motor 240 that is (via a rod that is not shown in the Figure), on the bottom side of the platform, coupled to a belt 250 (model of stepper motor may be: NEMA17-01, Neukirchen-Vluyn, Germany). The stepper motor causes a rotation of the rod, and moves the belt 250. There is a corresponding mechanism at the distal end of the platform, where the belt rotates a support bearing the sample (e.g., biological matter), here represented by a Petri dish 260, as a phantom wherein the robot may move. When the support rotates about an angle θ, the robot as such rotates with the biological matter.

Generally, the underlying principle of the magnetic trap system 1000 as shown in Fig. 3, is the following: A motion of the biological matter with the robot therein is caused by at least one of a motion of the linear stage 210 and the linear stage 220 and/ or the rotation due to the action of the stepper motor 240. Such motion is however counteracted as to the robot by the action of the magnetic field. Since the magnetic field exerted onto the robot would change due to the motion of the matter and of the robot therein, the robot is forced back to its original position in the magnetic trap. Since, on the other hand, the biological matter is not influenced by the magnetic field, the robot moves relative to, and inside of, the biological matter. The robot thus may be precisely navigated in the matter, by providing a controlled movement of the linear stage 210 and the linear stage 220, and of the stepping motor 240.

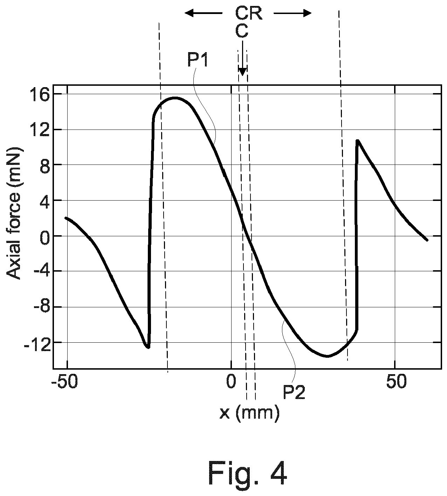

**Figure 4** illustrates an example of a magnetic force profile that may be predefined when designing the magnetic trap, the magnetic force profile enabling the implementation of the principle of the magnetic trap system 1000 explained in the preceding paragraph. The x-axis of the graph in Fig. 4 indicates the position of a robot along the longitudinal axis of the magnetic trap 100.

The y-axis of the graph in Fig. 4 indicates the force exerted onto the robot. The absolute values of the position and the magnetic force are not mandatory, the force profile works with respect to different quantities. The values indicated in Fig. 4 however best suit to the magnetic trap of the design shown in Figs. 2A and 2B and to a specific microscopic device as described hereinafter. Namely, the axial force is at an extremity more than 12 mN, for the matter penetration.

The force profile includes a portion CR (for "confinement region") in which the robot is attracted to the center, indicated here as C. Once in the CR, the robot cannot easily leave the CR, since in both directions, the force would shift it back to CR. The force profile is here partly linear in the confinement region, but may have a different graphical form. The force profile here specifically is shown to be strictly monotonically decreasing, namely linearly decreasing, in the confinement region CR from positive values of the force of a value of about 14 mN to minus 14 mN at the opposite side.

The value of more than 12 mN may be needed for being able to introduce the microscopic device into biological matter. Outside of the confinement region CR, at its border there is a steep (within not more than 1-4 mm width) change of the force profile from negative to positive on the left-hand side, and from positive to negative on the right-hand side. Hence, outside of the confinement region, the force pushes away from the trap.

Hence, the permanent magnet array is designed to create a strong magnetic force trap at a very small region CR in the space. This design optimization problem is formulated based on the magnetic dipole model. The magnetic field created by a magnetic dipole is

$$\mathbf{b} = \frac{\mu_0}{4\pi \|\mathbf{r}\|^3} \left( 3\hat{\mathbf{r}}\hat{\mathbf{r}}^{\mathrm{T}} - \mathbf{I} \right) \mathbf{m}$$

where $\mathbf{b} \in \mathbb{R}^3$ is the magnetic field, $\mu_0$ is the permeability of free space, $\mathbf{r} \in \mathbb{R}^3$ is the displacement vector of the point of interest from the center of the magnetic source, $\hat{\cdot}$ is the operator of vector normalization, I is the 3-by-3 identity

matrix, and $\mathbf{m} \in \mathbb{R}^3$ is the magnetic moment of the magnetic source. The magnetic force created on another magnetic source is

$$\mathbf{f} = \frac{3\mu_0}{4\pi\|\mathbf{r}\|^4}\left(\left(\hat{\mathbf{r}}^{\mathrm{T}}\mathbf{m}_r\right)\mathbf{m} + \left(\hat{\mathbf{r}}^{\mathrm{T}}\mathbf{m}\right)\mathbf{m}_r + \left(\mathbf{m}_r^{\mathrm{T}}\mathbf{m} - 5\left(\mathbf{r}^{\mathrm{T}}\mathbf{m}\right)\left(\mathbf{r}^{\mathrm{T}}\mathbf{m}_r\right)\right)\hat{\mathbf{r}}\right)$$

when the magnetic robot with magnetic moment $\mathbf{m}_r \in \mathbb{R}^3$ is located at r.

To create a magnetic force trap at the central axis of the array, one defines the force profile of Fig. 4. This force profile shows at least 12 mN of the magnetic force for the matter penetration, and crossing on the x-axis in CR for the force trap where the robot is automatically stabilized in the confinement region. This force profile becomes one of the constraints of the design optimization problem.

Additionally, one may define other constraints to restrict the radial force so that the robot does not drift away from the central axis with the help of matter resistance. The optimization goal here is to maximize the axial magnetic force. The configuration of the permanent magnets (e.g., the positions and orientations of the magnets) becomes the parameters for the optimization. All of this could be formulated in a nonlinear optimization routine as

$$\mathbf{x}^* = \arg\min_{\mathbf{x}} -|f_a(\mathbf{p}_1)|^2$$

subject to

$$f_a(\mathbf{p}_1) > 12 \text{ mN}$$

$$f_a(\mathbf{p}_2) < 0 \text{ mN}$$

$$\max(|f_r(\mathbf{p}_3)|) < 5 \text{ mN}$$

$$\frac{df_a(\mathbf{p}_4)}{dx} < 0$$

where $f_a(\mathbf{p})$ is the axial force evaluated at a point $\mathbf{p}$, $f_r(\mathbf{p})$ is the radial force evaluated at a point $\mathbf{p}$, $\mathbf{p}_1$ is the point on the axis in the workspace where the maximum force is intended (e.g., 20 mm away from the center of the array), $\mathbf{p}_2$ is the point on the other side of the axis restricting the force profile to cross the x-axis, $\mathbf{p}_3$ is the concatenated position vectors to represent distributed points (every point in a 2 mm grid) in the workspace to evaluate the multiple radial forces, x is the configuration of the permanent magnets including their positions and orientations assuming axisymmetric, and $\mathbf{p}_4$ is the concatenated point on the central axis of the array.

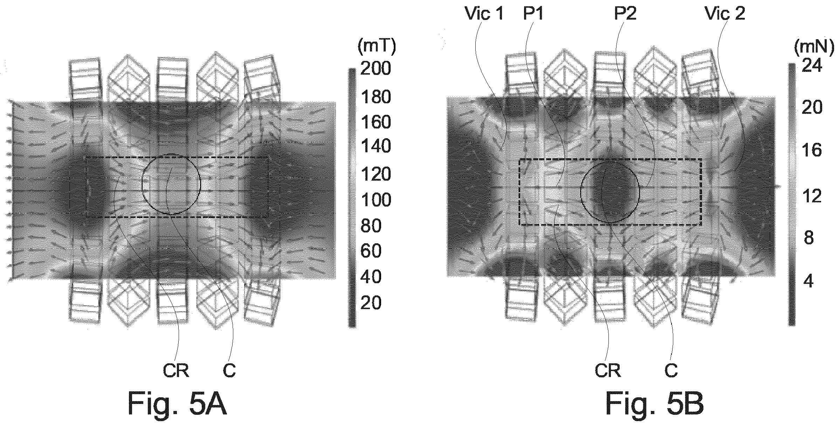

Here differentiation is forced to be negative to force the monotonically decreasing force profile as shown in Fig. 4. As a nonlinear solver, the interior point algorithm programmed in a commercial computing language (fmincon.m in MatLab, Mathworks, Inc., Massachusetts, United States) may be used to solve the formulated design optimization problem. The problem may be solved within about 150 iterations resulting in the configuration shown in Fig. 5A and 5B. Although the optimization is performed locally, the optimization converged to the final solution even with multiple random initial parameters, implying that the optimization is close to the global optimum.

The final design of the array may be verified by a commercial finite element analysis magnetic simulation tool (COMSOL Multiphysics 5.4, COMSOL Inc, Stockholm, Sweden) by placing the permanent magnets in the configuration achieved from the design optimization.

**Figure** 5A illustrates a distribution of the magnetic field obtained by the procedure explained above, with an indication as to the magnetic field strength as a shading degree. **Figure 5B** similarly illustrates a distribution of the magnetic force, with an indication as to the magnetic force strength as a shading degree.

As can be most easily gathered from Fig, 5A, there is a dipole field distribution in the interior of the magnetic field that corresponds to a dipole. One can thus identify the confinement region CR (here simplified as a rectangle, in broken lines). Outside of the CR, the magnetic field is the one of a reversed dipole. In other words, the arrows indicating the magnetic field direction enter the magnetic trap at the right-hand side to the left, and exit it at the left-hand side to the left as well. In the interior confinement region CR, the orientation of the magnetic field is pointing from left to right. The receptable zone RZ of fig. 5A may extend over the complete longitudinal length of the trap. E.g., the RZ may formed by the space surrounding the magnets of the trap. In the example of figure 2A, the RZ would be a tube with a length of 5 cm and a diameter of approx. 6 cm.

As can be gathered from Fig. 5B, the magnetic force acted upon a magnetic robot as caused by the magnetic field gradient is of a kind to repulse the robot both in the left-hand vicinity Vic 1 of the magnetic trap and its right-hand vicinity Vic 2 (see force arrows pointing to the outside). In contrast thereto, in the confinement region CR, the arrows at point P1 are to force the robot into the center region C, and the arrows at point P2 are to force the robot into the opposite direction, as well center region C. This is a consequence of the force profile in Fig, 3, see points P1 and P2 indicated therein.

In the present example, the magnetic field strength in the center region C is about 140 to 180 mT. The exerted force is between 4 and 8 mN. The magnetic field strength increases radially from the center region C to a value of 180 to 200 mT. The exerted force radially inside of the confinement region CR does not amount to more than 12 mN, even is preferably less than 8, more preferably less than 4 mN.

The magnetic field strength axially increases from both the left-hand and right-hand borders of the confinement region CR, where it has a value of about 50 or 60 mT, to the above-mentioned maximum value in the center region C. The longitudinal force has a maximum about the points P1 and P2.

The magnetic field and force distributions of Figs. 5A and 5B are obtained by orienting the permanent magnets in a specific way. As can be seen in Fig. 5A, the permanent magnets in the second and fourth row are oriented completely different than those in the first, third and fifth row. The algorithm used to define the distribution may be able to indicate to the constructor of the magnetic rap in detail how to exactly place the permanent magnets.

The robot when brought into vicinity Vic 1 (or Vic 2) of the magnetic trap does not itself enter the receptable zone RZ but is rather repulsed. Once the robot is forced by means of the mechanical stage 200 to the interior of the magnetic trap 100, i.e., to the receptable zone RZ, it is automatically forced into the confinement region CR. In an alternative, in case the employed magnets are not permanent magnets but electromagnets which can be turned on and off by respective control means. The robot may thus already be located in the matter in a desired location and the matter may be positioned relative to the CR, preferably in the center C of the CR. Then, the electromagnet may be turned on, thus providing the magnetic trap holding the robot in its current position C or forcing the robot to move to the position C.

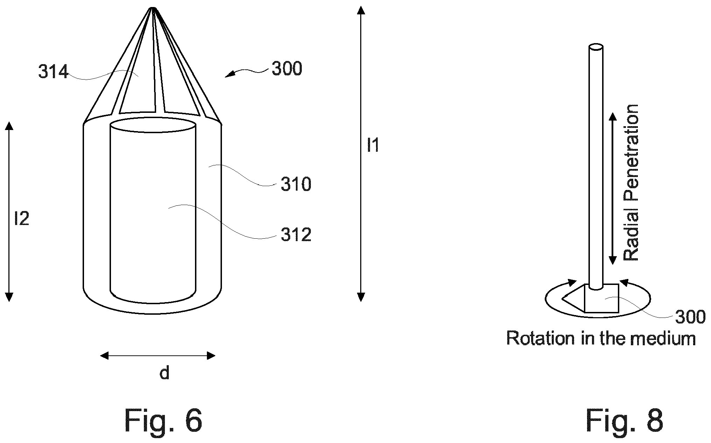

**Figure 6** illustrates an exemplary shape of a robot 300 that may be used in the magnetic trap system according to an embodiment.

The robot 300 may comprise a main body 310 which may be cylindrical and in which a likewise cylindrical permanent magnet 312 may be housed. The permanent magnet 312 may be fully or at least partly include NdFeB. The robot 300 may further include a tip 314, which may be conical. By suitably shaping the tip, the robot 300 when magnetic force acts upon it (i.e., the permanent magnet 312), the tip enhances the facility of moving by shifting obstacles to the side.

The entire length 11 of the robot 300 may be about 3 mm (between 1.5 and 5 mm). The length 12 of the main body may be about 2 (between 1.5 and 2.5 mm). The permanent magnet 312 may have the same length, or at least a length of between 95 % and 99.8 % of the cylinder length. The diameter d of the cylindrical main body 310 may be about 1 mm (between 0.5 mm and 2 mm). The permanent magnet 312 may have the same diameter, or at least a diameter of between 95 % and 99.8 % of the cylinder diameter. The conical tip 314 has a radius r of about 50 $\mu$m (between 35 and 65 $\mu$m) at its distal end.

The robot 300 may have a principal axis extending in the longitudinal direction. This may be defined by indicating an aspect ratio of length 11 to diameter d to be more than 1.5 and preferably more than 2. A considerable stability when navigating may be obtained if the aspect ratio is less than 8, preferably less than 6, further preferably less than 4. Rather than using the aspect ratio of length l1 to diameter d, one may define an aspect ratio of length l2 to diameter d, for instance as having a value between 2 and 3.

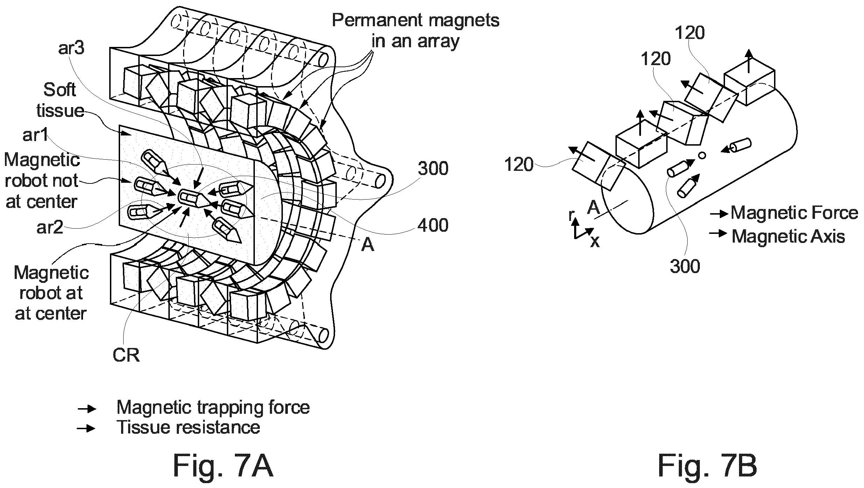

**Figure 7A** illustrates an exemplary situation of the robot 300 in the magnetic trap 100, namely in confinement region CR. The robot 300 is embedded in biological matter 400. The magnetic trap's magnetic field forces the robot to remain (be confined in) the confinement region CR by exerting axial forces as symbolized by arrows ar1 and ar2, and others. On the other hand, the biological matter itself has a viscosity that impedes the robot to move radially. The viscosity exerts a counterforce as symbolized by arrow ar3 and others. Due to the fact that the exerted force that would be able to shift the robot 300 radially outside of the confinement region CR does not amount to more than 6 to 8 mN (as shown in Fig. 5B), the robot 300 would as a result not be shifted radially outside of the confinement region CR. It is a constraint that may be imposed upon the entire system that with respect to the following system properties:

the robot has a specific weight,

the permanent magnet in the robot has specific properties,

the trap has a specific field strength and

field gradient,

the properties are matched to the properties of the matter to be navigated in, in a manner that the robot is not radially moved in the magnetic trap's confinement region CR. This protects the biological matter from an "escaping robot". The above values of 6 to 8 mN are suited to the above-described robot. Other values might be required and be suitable if the robot had a different shape, weight and magnet.

**Figure 7B** illustrates as well an exemplary situation of the robot 300 in the magnetic trap. The arrows extending from the permanent magnets 120 symbolize magnetic field orientation. It is visible that the specific orientation of the permanent magnets 120 assists - further to the effect of matter viscosity - in aligning the robot 300 to the longitudinal axis, with an exactitude of some angular degrees, e.g., of 10 °. By such aligning, the navigation can be done more reliably, since the orientation of the robot is not an issue.

**Figure 8** illustrates theoretical movements of the robot 300, namely a rotation in the biological matter (the medium), which might be desired, and a radial penetration of the matter, which is undesired.

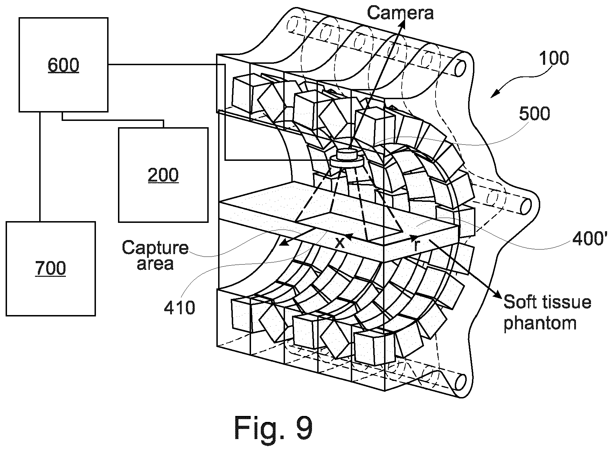

**Figure 9** illustrates how a robot in the confinement region of the magnetic trap of Fig. 1A/1B can be monitored: In the example, the magnetic trap 100 comprises a camera 500 (e.g., model: YC225-P/FBA, CORPRIT, China) in its interior, i.e. the receptable zone RZ. The sample 400' (soft matter, or a respective phantom) can then be subject to an imaging. The camera 500 may symbolize any kind of imaging technique, not only optical, but as well infrared, ultraviolet, or X-Ray fluoroscopy (for instance, model: XPERT 80, KUBTEC, Stratford CT, USA), or sound and/or ultrasound. The imaging means may be placed at least partly outside of the magnetic trap 100. Then, one may omit one, two, three or more of the numerous (e.g. of 95) permanent magnets 120 of the magnetic trap 100 in order to allow radiation or sound to get through to the receptable zone RZ. Such omission generally does only have a minor influence on the magnetic field distribution.

The imaging system can capture an imaging area 410 of the sample 400'. The imaging makes sense in order to monitor the robot in matter. The imaging system may be coupled to a computer system 600 that acts as a controller for the mechanical stage 200 (for instance: Robot Operating System (ROS Melodic) which runs in Linux operating system (Ubuntu 18.04)). The computer system 600 may include image recognition software to be able to detect the position of the robot 300 in the sample 400'. Then, respective (feedback) control signals can be sent to the mechanical stage 200 in order to have the robot 300 move along a desired path. The system may include an input-output device 700 for an operator to be able to view images taken on a display thereof, and to react thereon by inputting control orders. Input may be provided through a wireless gamepad (model: F710, Logitech, Newark CA, USA).

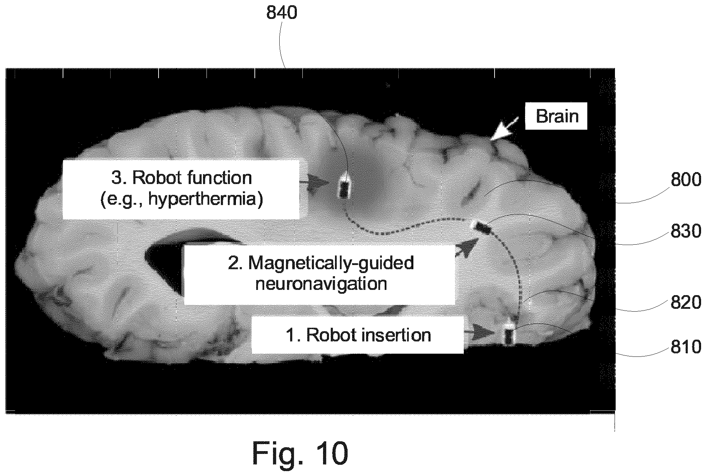

**Figure 10** illustrates a brain to explain the steps of a method of navigating a robot therein, and a method of treating the brain (not claimed herein).

The robots that may be usable in a method of navigating as explained above may - as commonly - serve a specific treatment purpose.

For example, a robot that can navigate inside brain could treat brain cancer by e.g. releasing certain anti-cancer medication at desired locations without creating large surgical openings in skull and brain matters. Recent attempts to enable remotely-actuated miniature robots to navigate inside matters have aimed for blood-clot removal and matter drilling.

Hence, the robots may in principle look like the robot 300 described above with respect to Fig 6, but may be equipped with something further, like a vessel for a medication, a mechanical unit for mechanically acting upon matter, or a heat producing unit (using chemicals or electricity). There may in particular be a source of electrical energy (such as a battery) included. There may as well be communication means (for a wireless robot control), such as electromagnetic transceivers, and the like. There may as well be a microprocessor or microcontroller for the overall control of the robot's units.

The method of navigating has been tested with a sample of porcine brain, shown at 800 in Fig. 10 ex vivo. The robot 300 is inserted at opening 810 into the brain 800. There is a natural pathway 820 in the brain, such as an artery or vein, along which the robot may be navigated, using the above-described system and method. The robot is shown at 830 in the pathway 820 at an intermediate position. The robot is finally navigated to a target position 840, where the robot may be able to provide the treatment of the matter.

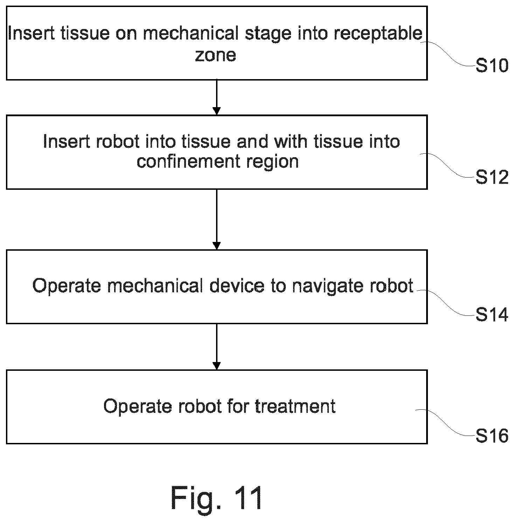

**Figure 11** is a flow diagram that illustrates the steps of a method of medically or surgically treating a subject by navigating a robot in the subject's matter, the navigating in accordance with the present subject matter.

Starting with placing the matter on the respective support of the platform 230 and introducing the platform 230 into the receptable zone RZ at step S10, inserting the robot into matter and with the matter into the confinement region CR follows at step S12, and the mechanical stage 200 is operated at step S14 with the effect that the matter moves relative to the magnetic trap 100, where there is a counteraction of the robot under effect of the magnetic fields, as explained above. The robot arrives at its target position and is itself wirelessly operated in step S 16 for treatment (release of heat, and the like). Steps S10 and S12 might be interchanged, i.e. the robot first inserted into the matter before placing it into the receptable zone RZ. The method claimed herein does not include steps S12 and S16.

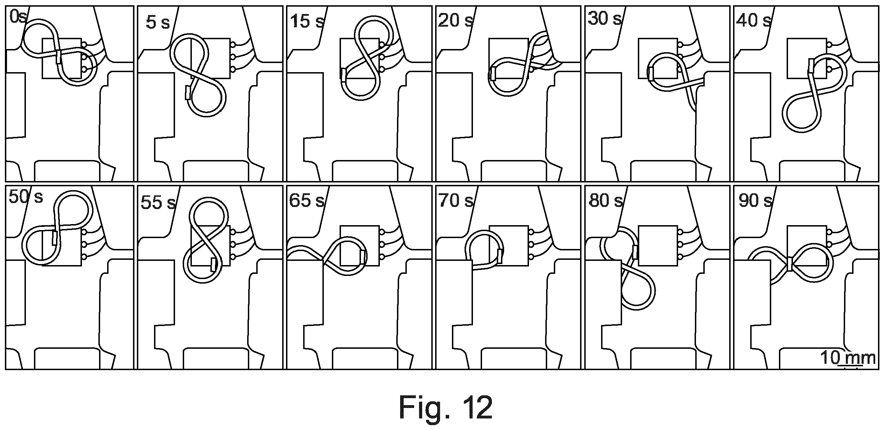

**Figure 12** shows a sequence of fluoroscopic images illustrating navigating of a robot in biological matter.

The robot has here been navigated a matter phantom to undergo the path of the infinity symbol (lying 8).

As will be appreciated by one skilled in the art, aspects of the present invention may be embodied as a apparatus, method, computer program or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon. A computer program comprises the computer executable code or "program instructions".

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Generally, the program instructions can be executed on one processor or on several processors. In the case of multiple processors, they can be distributed over several different entities like clients, servers etc. Each processor could execute a portion of the instructions intended for that entity. Thus, when referring to a system or process involving multiple entities, the computer program or program instructions are understood to be adapted to be executed by a processor associated or related to the respective entity.

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

**Claims**

1. A magnetic trap system (1000) comprising:

   - a microscopic device (300), comprising a principal axis extending in a longitudinal direction;
   - a trap (100) for magnetically confining the microscopic device (300) in a confinement region (CR);
   - a receptable zone (RZ) within the trap (100) for receiving biological matter (400, 800), the receptable zone (RZ) comprising the confinement region (CR);
   - a mechanical device (200) for providing a relative movement between the receptable zone (RZ) and the microscopic device (300);
   wherein the trap (100) is hollow about a longitudinal axis (A) and comprises the receptable zone (RZ);
   wherein the trap (100) is configured to provide a magnetic field gradient configured to confine the microscopic device to the confinement region (CR) of the trap (100); and
   wherein the orientation of the magnetic field in the confinement region (CR) of the trap (100) is configured to align the principal axis of the microscopic device (300) in the confinement region (CR) with the longitudinal axis (A) of the confinement region or with an axis that does not deviate more than 30° from the longitudinal axis (A) of the confinement region (CR).

2. The system (1000) of claim 1, wherein the microscopic device (300) has a main tubular, preferably cylindrical, body (310) and a tip (314), preferably conical tip, that extends from the main body (310).

3. The system (1000) of claim 2, wherein the microscopic device (300) has an aspect ratio of its overall length (11) to a diameter (d) of the main body (310) of between 0.1 and 1000, preferably between 0.5 and 5, more preferably of between 1.5 and 3.5, still more preferably of between 2.3 and 2.8.

4. The system (1000) of any one of the preceding claims, wherein the microscopic device (300) has a length (11) of between 0.1 and 100 mm and a width or diameter (d) of between 0.001 and 5.0 mm.

5. The system (1000) of any one of the preceding claims, wherein the microscopic device is configured as a robot (300) for medical or surgical treatment or diagnosis.

6. The system (1000) of any one of the preceding claims, wherein the trap (100) comprises a plurality of permanent magnets (120) adapted for providing the optimal trapping magnetic fields.

7. The system (1000) of any one of the preceding claims, wherein the trap (100) is configured to provide a magnetic field within the trap that has at least one of the following properties:

   a) the magnetic field vectors in the center of the confinement region (CR) are parallel to the longitudinal axis (A) or are parallel to an axis deviating not more than 30°, preferably not more than 15°, from the longitudinal axis (A),
   b) the magnetic field strength parallelly to the longitudinal axis (A) increases from both the left-hand and right-hand borders of the receptable zone (RZ), wherein it has preferably a value of between 1 mT and 100 mT, to a value defined in the confinement region (CR),
   c) the value of the magnetic field strength in the confinement region (CR) is between 1 mT to 500 mT,
   d) the magnetic field strength radially increases from the center of the confinement region (CR) from a minimum value to a value not more than 15 % of the minimum value,
   e) the magnetic field strength increases radially from the center of the confinement region (CR) to a maximum value of 10 mT to 500 mT,
   f) parallel to the longitudinal axis (A) the magnetic field gradient in the confinement region (CR) of the trap (100) has a value of between zero in the center of the confinement region and of between 0.1 and 30 T/m, preferably between 6 and 8 T/m G in the immediate vicinity of the border of the confinement region,
   g) the magnetic field gradient at the border of an confinement region (CR) has an absolute value of more than 5 T/m urging the microscopic device to the confinement region (CR),
   h) the absolute value of the magnetic field gradient inside the confinement region (CR) monotonically decreases, preferably strictly monotonically decreases, still more preferably linearly decreases, from a border of the confinement region (CR) to a minimum value in the center of the confinement region (CR).

8. The system (1000) of any one of the preceding claims, wherein the trap (100) is configured to provide the magnetic field gradient using a first magnetic field and wherein the microscopic device is configured to provide a second magnetic field, wherein the first and second magnetic fields comprise at least one of the following properties:

   a) the absolute value of the radial force magnetically exerted onto the microscopic device (300) in the confinement region (CR) is less than 10 mN, preferably less than 4 mN, more preferably less than 2 mN,
   b) the absolute value of the axial force magnetically exerted onto the microscopic device (300) parallel to the longitudinal axis (A) in the confinement region (CR) is in between 0 in the center of the confinement region and F in the immediate vicinity of the border of the confinement region, F being in between 4-24 mN, preferably 12-16 mN.

9. The system (1000) of any one of the preceding claims, wherein the trap (100) and the microscopic device (300) are matched to each other in that the confinement region (CR) has a length of between 1 time to 500 times, preferably of between 15 times and 25 times, the length of the microscopic device (300) along its principal axis and/or in that the confinement region (CR) has a width of between 1 time and 500 times, preferably between 13 times and 18 times the width of the microscopic device (300) perpendicular to its principal axis.

10. The system (1000) of any one of the preceding claims, further comprising a medical imaging device (500), preferably an X-ray fluoroscopic or ultrasound imaging device, adapted for monitoring the microscopic device in the confinement region (CR).

11. The system (1000) of claim 10, including a controller (600) for controlling the mechanical device (200) for performing the relative movement between the receptable zone (RZ) and the microscopic device (300), wherein the controller (600) is configured to receive image data of images taken by the imaging device, to analyze the data, and to control the mechanical device (200) in dependence of the data and a result of the analysis.

12. The system (1000) of any one of the preceding claims, wherein the relative movement provided by the mechanical device (200) is anyone of a longitudinal motion, a radial motion, and a rotation with respect to the longitudinal axis (A) of the trap (100).

13. A method of navigating a microscopic device in biological matter matter, the method comprising:

   providing a magnetic trap system (1000), the magnetic trap system (1000) comprising:

   a) a microscopic device (300), comprising a principal axis extending in a longitudinal direction;

b) a trap (100) for magnetically confining the microscopic device (300) in a confinement region (CR);
c) a receptable zone (RZ) for receiving biological mattermatter (400, 800), the receptable zone (RZ) comprising the confinement region (CR);
d) a mechanical device (200) for providing a relative movement between the receptable zone (RZ) and the microscopic device (300);

wherein the trap (100) is hollow about a longitudinal axis (A) and comprises the receptable zone (RZ);
wherein the trap (100) is configured to provide a magnetic field gradient configured to confine the microscopic device (300) to the confinement region (CR) of the trap; and

wherein the orientation of the magnetic field in the confinement region (CR) of the trap is configured to align the principal axis of the microscopic device (300) in the confinement region (CR) with the longitudinal axis (A) of the confinement region (CR) or with an axis that does not deviate more than 30° from the longitudinal axis (A) of the confinement region (CR).

14. The method of claim 13, the method further comprising:

◦ positioning (S212) the biological mattermatter (400, 800) with a microscopic device inserted therein in the receptable zone (RZ) ;
◦ operating (S214) the mechanical device (200) for performing the relative movement between the receptable zone (RZ) and the microscopic device (300) in order to cause the microscopic device (300) to undergo navigation in the biological mattermatter (400,800).

15. The method of claim 13 or 14, further comprising providing an imaging device (500), preferably an X-ray fluoroscopic or ultrasound imaging device, adapted for monitoring the microscopic device in the confinement region (CR), the method further comprising receiving a navigation path, the navigation path describing a desired relative movement of the microscopic device relative to the biological mattermatter, wherein operating (S214) the mechanical device (200) for performing the relative movement between the receptable zone (RZ) and the microscopic device (300) is performed in order to cause the microscopic device (300) to undergo navigation in accordance with the navigation path in the biological mattermatter (400,800), the method further comprising during the navigation:

◦ receiving image data of images taken by the imaging device from the confinement region,
◦ receiving a desired spatial location of the microscopic device relative to the biological mattermatter in accordance with the navigation path,
◦ analyzing the data for obtaining an actual spatial location of the microscopic device relative to the biological mattermatter, and in case of a mismatch between the actual location and the desired location, control the mechanical device (200) in dependence of the data and a result of the analysis for correcting the actual spatial location of the microscopic device, the correcting resulting in a matching of the actual spatial location of the microscopic device with the desired spatial location.

16. The method of any one of claims 13 to 15, the magnetic field of the trap and the microscopic device being adapted to the biological mattermatter such that in the confinement region (CR) the radial magnetic force acting onto the microscopic device is smaller or equal than the friction force acting between the mattermatter and the microscopic device in the radial direction, while in the longitudinal direction the magnetic force acting onto the microscopic device has a value in between zero and a value which is larger than the friction force acting between the mattermatter and the microscopic device.

17. The method of claim 16, wherein the radial magnetic force acting onto the microscopic device is to a factor of between 0.25 and 0.9, preferably of between 0.7 and 0.8, smaller or equal than the friction force acting between the mattermatter and the microscopic device in the radial direction.

18. The method of claim 16 or claim 17, wherein the magnetic force acting onto the microscopic device longitudinal direction is to a factor of between 1.1 and 1.8, preferably of between 1.2 and 1.3, larger than the friction force acting between the mattermatter and the microscopic device.

19. The method of any one of claims 12 to 18, wherein the method further comprises providing the trap, the trap comprising a plurality of magnets, wherein the providing comprises determining the relative arrangement of the magnets to each other, the determining being performed employing a numerical nonlinear optimization solver employing a magnetic dipole model.

20. A computer program product, in particular a computer readable medium, the computer program product carrying computer executable code for execution by a processor controlling the system of claim 1, wherein execution of the instructions cause the processor to control the mechanical device (200) for performing the relative movement between the receptable zone (RZ) and the microscopic device (300).

Fig. 1

Fig. 2A

Fig. 2B

Fig. 3

Fig. 4

Fig. 5A

Fig. 5B

Fig. 6

Fig. 8

314
300
310
312

l1
l2
d

Radial Penetration

300

Rotation in the medium

ar3

Soft tissue

ar1

Magnetic robot not at center

ar2

Magnetic robot at at center

CR

Permanent magnets in an array

300

400

A

→ Magnetic trapping force
→ Tissue resistance

Fig. 7A

120

120

120

A

r

x

→ Magnetic Force
→ Magnetic Axis

300

Fig. 7B

EP 4 124 314 A1

Fig. 9

Fig. 10

Insert tissue on mechanical stage into receptable zone —— S10

Insert robot into tissue and with tissue into confinement region —— S12

Operate mechanical device to navigate robot —— S14

Operate robot for treatment —— S16

## Fig. 11

Fig. 12

Europäisches
Patentamt

European
Patent Office

Office européen
des brevets

## EUROPEAN SEARCH REPORT

Application Number

EP 21 18 7691

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/022835 A1 (KIMURA ATSUSHI [JP] ET AL) 28 January 2010 (2010-01-28) | 1,2,5,7, 20 | INV. A61B34/30 |
| Y | * paragraphs [0015] – [0025], [0036], [0084] – [0096], [0112], [0148], [0172], [0232], [0242], [0243]; figures 1–18 * | 3,4,6, 8–12 | A61B34/00 ADD. A61B1/00 |
| Y | US 2011/282165 A1 (KAWANO HIRONAO [JP] ET AL) 17 November 2011 (2011-11-17) * paragraph [0032] * * paragraph [0048] * * paragraph [0088] – paragraph [0089] * * figures 1–17 * | 3,4,8,9 | |
| Y | US 2021/052190 A1 (KISELYOV ALEX [US] ET AL) 25 February 2021 (2021-02-25) * paragraphs [0004] – [0021], [0032], [0033], [0044] – [0046], [0053], [0057], [0068] – [0079], [0088]; figures 1–25 * | 6,10–12 | |
| A | MANAMANCHAIYAPORN LALIPHAT ET AL: "The HyBrid system with a large workspace towards magnetic micromanipulation within the human head", 2017 IEEE/RSJ INTERNATIONAL CONFERENCE ON INTELLIGENT ROBOTS AND SYSTEMS (IROS), IEEE, 24 September 2017 (2017-09-24), pages 401-407, XP033265958, DOI: 10.1109/IROS.2017.8202186 [retrieved on 2017-12-13] * page 401 – page 407 * | 1–12,20 | TECHNICAL FIELDS SEARCHED (IPC) A61B |

-/--

The present search report has been drawn up for all claims

| Place of search | Date of completion of the search | Examiner |
|---|---|---|
| The Hague | 23 December 2021 | Viidebaum, Mikk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

**EP 21 18 7691**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/219207 A1 (MAX PLANCK GESELLSCHAFT [DE]) 21 November 2019 (2019-11-21)<br>* page 3 – page 13 *<br>* page 16 – page 19 *<br>* page 27 – page 28 *<br>* figures 1-17 *<br>----- | 1-12,20 | |
| A | KR 2014 0026958 A (UNIV NAT CHONNAM IND FOUND [KR]) 6 March 2014 (2014-03-06)<br>* abstract; figures 2-7 *<br>----- | 1-12,20 | |

**TECHNICAL FIELDS SEARCHED (IPC)**

The present search report has been drawn up for all claims

| Place of search | Date of completion of the search | Examiner |
|---|---|---|
| The Hague | 23 December 2021 | Viidebaum, Mikk |

EPO FORM 1503 03.82 (P04C01)

## ANNEX TO THE EUROPEAN SEARCH REPORT
## ON EUROPEAN PATENT APPLICATION NO.

EP 21 18 7691

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2021

| Patent document cited in search report | | Publication date | Patent family member(s) | | Publication date |
|---|---|---|---|---|---|
| US 2010022835 | A1 | 28-01-2010 | CN 101484063 A | | 15-07-2009 |
| | | | EP 2036483 A1 | | 18-03-2009 |
| | | | JP 4898807 B2 | | 21-03-2012 |
| | | | JP WO2008004497 A1 | | 03-12-2009 |
| | | | US 2010022835 A1 | | 28-01-2010 |
| | | | WO 2008004497 A1 | | 10-01-2008 |
| US 2011282165 | A1 | 17-11-2011 | CN 102227187 A | | 26-10-2011 |
| | | | EP 2353489 A1 | | 10-08-2011 |
| | | | JP 4903899 B2 | | 28-03-2012 |
| | | | JP WO2010061894 A1 | | 26-04-2012 |
| | | | US 2011282165 A1 | | 17-11-2011 |
| | | | WO 2010061894 A1 | | 03-06-2010 |
| US 2021052190 | A1 | 25-02-2021 | EP 3787997 A1 | | 10-03-2021 |
| | | | JP 2021522960 A | | 02-09-2021 |
| | | | US 2021052190 A1 | | 25-02-2021 |
| | | | WO 2019213362 A1 | | 07-11-2019 |
| WO 2019219207 | A1 | 21-11-2019 | EP 3793462 A1 | | 24-03-2021 |
| | | | JP 2021529568 A | | 04-11-2021 |
| | | | US 2021228298 A1 | | 29-07-2021 |
| | | | WO 2019219207 A1 | | 21-11-2019 |
| KR 20140026958 | A | 06-03-2014 | NONE | | |

EPO FORM P0459

For more details about this annex : see Official Journal of the European Patent Office, No. 12/82